# FASCICULE DE BREVET EUROPEEN

(11) **EP 0 987 011 B1**
(45) Date de publication et mention de la délivrance du brevet: **17.07.2002**
(21) Numéro de dépôt: 99401738.2
(22) Date de dépôt: 09.07.1999
(51) Int. Cl.: A61K 7/48

(54) **Composition cosmétique et/ou dermatologique contenant de l'acide salicylique ou un dérivé d'acide salicylique et son utilisation**
Kosmetische und/oder dermatologische Zusammensetzung enthaltend Salicylsäure oder ihre Derivate und ihre Verwendung
Cosmetic and/or dermatological composition containing salicylic acid or its derivatives and its use

(30) Priorité: 17.08.1998 FR 9810472
(43) Date de publication de la demande: 22.03.2000
(73) Titulaire: L'OREAL, 75008 Paris (FR)
(72) Inventeur: Touzan, Philippe, 75012 Paris (FR); Delambre, Patricia, 94480 Ablon/S/Seine (FR)
(74) Mandataire: Rasson, Catherine

(56) Documents cités:
- EP-A- 0 378 936
- WO-A-97/44049
- WO-A-98/10741
- WO-A-98/22083
- FR-A- 2 755 366
- US-A- 5 531 993

## Description

L'invention se rapporte à une composition cosmétique et/ou dermatologique sous forme d'émulsion huile-dans-eau à base d'acide salicylique et/ou d'un dérivé d'acide salicylique, contenant un ester de glucose ou d'alkylglucose et un éther oxyéthyléné de glucose et/ou d'alkylglucose. Elle se rapporte aussi à l'utilisation de cette composition pour lutter, en douceur, contre le vieillissement de la peau du visage et/ou du corps humain, y compris le cuir chevelu, et pour lutter contre l'acné et/ou les désordres cutanés.

Il est connu d'utiliser les bêta-hydroxyacides et notamment l'acide salicylique et ses dérivés comme agents kératolytiques pour traiter l'acné et comme agents d'antivieillissement dans des compositions cosmétiques et/ou dermatologiques. Ainsi, le document WO-A-93/10756 décrit l'utilisation d'une composition à base d'acide salicylique pour traiter les rides et les documents FR-A-2 581 542 et EP-A-378 936 décrivent l'utilisation de dérivés d'acide salicylique pour traiter l'acné et les signes du vieillissement.

L'acide salicylique et ses dérivés sont d'un grand intérêt, étant donné leurs effets biologiques sur la peau, notamment sur les principaux signes cliniques du vieillissement cutané que sont les ridules et rides, la désorganisation du "grain" de la peau, la modification du teint de la peau et la perte de fermeté et de tonicité de la peau. En outre, les dérivés d'acide salicylique présentent l'avantage par rapport à l'acide salicylique, d'avoir une activité kératolytique supérieure et une activité bactériostatique efficace.

Malheureusement, l'utilisation de ces actifs pose un problème dans la mesure où ils peuvent provoquer des picotements, des démangeaisons, des tiraillements après leur application, pouvant conduire à un important inconfort. L'utilisation de ces composés pour les utilisateurs à peau sensible est donc souvent rédhibitoire. En outre, il est parfois nécessaire d'ajouter à ces composés, des solvants tels que l'octyldodécanol, pour les solubliser, ce qui peut ajouter à l'inconfort de la composition les contenant.

Aussi, il subsiste le besoin d'une composition cosmétique et/ou dermatologique à base de tels composés, qui ne présente pas les inconvénients de l'art antérieur.

La demanderesse a trouvé de façon inattendue que l'on peut augmenter de façon significative la tolérance de l'acide salicylique et de ses dérivés en les incorporant dans une émulsion huile-dans-eau (H/E) ayant un pH égal ou supérieur à 4 et contenant comme tensioactifs un mélange d'ester de glucose ou d'alkylglucose et d'éther oxyéthyléné de glucose ou d'alkylglucose.

La présente invention a donc pour objet une composition cosmétique et/ou dermatologique sous forme d'une émulsion huile-dans-eau, caractérisée en ce qu'elle a un pH allant de 4 à 7 et en ce qu'elle contient (i) de l'acide salicylique et/ou au moins un dérivé d'acide salicylique, (ii) au moins un ester d'acide gras et de glucose et/ou d'alkylglucose, et (iii) au moins un éther oxyéthyléné d'acide gras et de glucose et/ou d'alkylglucose.

L'association d'un ester de (alkyl)glucose et d'un éther oxyéthyléné de (alkyl)glucose permet d'obtenir une composition parfaitement tolérée lorsqu'elle est appliquée sur la peau du visage et/ou du corps humain. Cette association est, selon un mode préfère de réalisation de l'invention, utilisée comme seul tensioactif.

La chaîne grasse dans l'ester d'acide gras et de (alkyl)glucose et celle dans l'éther oxyéthyléné d'acide gras et de (alkyl)glucose comportent de préférence de 8 à 30 atomes de carbone et plus particulièrement de 10 à 22 atomes de carbone.

Comme ester d'acide gras et de glucose ou d'alkylglucose, on peut citer en particulier les esters gras de méthylglucoside et plus spécialement le diester de méthylglucoside et d'acide oléique (nom CTFA : Methyl glucose dioleate) ; l'ester mixte de méthylglucoside et du mélange acide oléique / acide hydroxystéarique (nom CTFA : Methyl glucose dioleate/hydroxystearate) ; l'ester de méthylglucoside et d'acide isostéarique (nom CTFA : Methyl glucose isostearate) ; l'ester de méthylglucoside et d'acide laurique (nom CTFA : Methyl glucose laurate) ; l'ester de méthylglucoside et d'acide isostéarique (nom CTFA : Methyl glucose isostearate) ; le mélange de monoester et de diester de méthylglucoside et d'acide isostéarique (nom CTFA: Methyl glucose sesqui-isostearate) ; le mélange de monoester et de diester de méthylglucoside et d'acide stéarique (nom CTFA : Methyl glucose sesqui-stearate) et en particulier le produit commercialisé sous la dénomination Glucate SS par la société AMERCHOL. On peut aussi utiliser un mélange de ces esters.

De préférence, l'ester d'acide gras et de glucose ou d'alkylglucose est introduit dans la phase huileuse de l'émulsion. Il est utilisé en une quantité satisfaisante pour assurer le résultat escompté. Ainsi, cette quantité peut aller par exemple de 0,1% à 10% en poids et de préférence de 1% à 3% en poids par rapport au poids total de la composition.

L'éther oxyéthyléné d'acide gras et de glucose ou d'alkylglucose comporte de préférence de 10 à 100 groupements oxyéthylénés (ou moles d'oxyde d'éthylène) et mieux de 20 à 40 groupements oxyéthylénés. Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom CTFA : PEG-20 methyl glucose distearate) tel que le produit commercialisé sous la dénomination Glucam E-20 distearate par la société AMERCHOL ; l'éther de polyéthylène glycol du mélange de monoester et de diester de méthyl glucose et d'acide stéarique à environ 20 moles d'oxyde d'éthylène (nom CTFA : PEG-20 methyl glucose sesquistearate) et en particulier le produit commercialisé sous la dénomination Glucamate SSE-20 par la société AMERCHOL et celui commercialisé sous la dénomination Grillocose PSE-20 par la société GOLDSCHMIDT. On peut aussi utiliser un mélange de ces éthers oxyéthylénés.

De préférence, l'éther oxyéthyléné de (alkyl)glucose est introduit dans la phase aqueuse de l'émulsion. Il est utilisé en une quantité satisfaisante pour assurer le résultat escompté. La quantité d'éther(s) oxyéthyléné(s) d'acide gras et de (alkyl)glucose peut aller par exemple de 0,1% à 10% en poids et de préférence de 1% à 3% en poids par rapport au poids total de la composition.

La composition de l'invention peut comprendre de l'acide salicylique et/ou un ou plusieurs dérivés de l'acide salicylique.

Comme dérivés de l'acide salicylique, on peut utiliser tout dérivé susceptible d'être utilisé dans une composition cosmétique et/ou dermatologique, et notamment les dérivés d'acide salicylique de formule (I) ou un sel d'un tel dérivé : 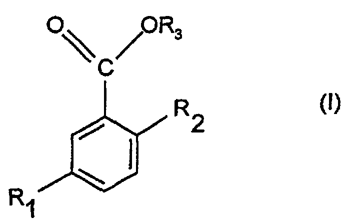 dans laquelle :
R₁ représente l'hydrogène ou une chaîne aliphatique, alcoxy, ester ou cétoxy, saturée, linéaire, ramifiée ou cyclisée, une chaîne insaturée portant une ou plusieurs doubles liaisons conjuguées ou non, ces chaînes comportant de 1 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;
R₂ représente un groupement hydroxyle ou une fonction ester de formule (II) : 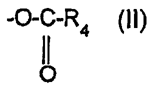 où R₄ représente un groupement aliphatique saturé ou un groupement alcényle ayant de 1 à 18 atomes de carbone ;
R₃ représente l'hydrogène ou une chaîne linéaire ou ramifiée, saturée ou insaturée, ayant de 2 à 30 atomes de carbone, comportant éventuellement un ou plusieurs substituants. La chaîne peut être choisie en particulier parmi les radicaux alkyle et alcényle comportant de 2 à 30 atomes de carbone, éventuellement substitués. Le substituant peut être en particulier un radical hydroxylé.

Quand R₃ est l'hydrogène, on peut aussi utiliser des sels des acides de formule (I), et en particulier des sels obtenus par salification avec une base.

Comme base susceptible de salifier les dérivés de l'acide salicylique de formule (I), on peut citer les bases minérales comme les hydroxydes de métaux alcalins (hydroxydes de sodium et de potassium) ou les hydroxydes d'ammonium ou mieux encore les bases organiques telles que les amines organiques primaires, secondaires, tertiaires ou cycliques et plus spécialement les acides aminés. A titre d'exemple de bases, on peut citer la glycine, la lysine, l'arginine, la taurine, l'histidine, l'alanine, la valine, la cystéïne, la trihydroxy-méthylaminométhane (TRISTA), la triéthanolamine.

Selon un mode particulier de réalisation de l'invention, on utilise dans la composition de l'invention un dérivé de formule (I) où le radical R₁ comporte au moins 4 atomes de carbone. Il est par exemple formé d'un radical alkyle ou alcoxy linéaire saturé ayant de 4 à 11 atomes de carbone.

Comme dérivés de formule (I) dans laquelle R₂ est un hydroxyle et R₃ est l'hydrogène, on peut citer notamment les acides n-octanoyl-5-salicylique (nom CTFA : Capryloyl Salicylic Acid), n-décanoyl-5-salicylique, n-dodécanoyl-5-salicylique, n-octyl-5-salicylique, n-heptyloxy-5-salicylique, 5-tert-octylsalicylique, 5-butoxysalicylique, 5-éthoxysalicylique, 5-méthoxysalicylique, 5-propoxysalicylique, 5-méthylsalicylique, 5-éthylsalicylique, 5-propylsalicylique, ces acides étant éventuellement salifiés par une base.

Quand R₁ représente l'hydrogène et R₂ un groupement hydroxyle, le dérivé de formule (I) est un ester d'acide salicylique. Il s'agit de préférence d'esters d'alcools gras tels que les esters d'alcools dodécylique, hexadécylique, stéarylique, cétylique, myristylique, linoléylique, octylique, oléique, tridécylique, ou également d'esters d'alcools butylique, propylique, éthylique, ou encore les esters de polyols tels que les esters de propylène glycol, de butylène glycol, d'éthylène glycol ou de glycérol, ou des mélanges de ces esters. Il peut s'agir notamment du salicylate de cétyle, du salicylate de dodécyle et du salicylate de tridécyle.

L'acide salicylique et ses dérivés sont utilisés dans la composition selon la présente invention, en quantité suffisante pour atteindre le résultat cosmétique ou dermatologique escompté. La quantité en acide salicylique et/ou dérivé(s) d'acide salicylique va de préférence de 0,001 à 10 % en poids et mieux de 0,05 à 5 % en poids par rapport au poids total de la composition.

LLa composition selon l'invention a un pHallant de 4 à 7 et de préférence de 4 à 6. Il s'ensuit une grande compatibilité de l'émulsion de l'invention vis-à-vis de la peau.

La composition de l'invention est destinée à une application topique et comprend de manière appropriée un milieu physiologiquement acceptable. Par milieu physiologiquement acceptable, on entend un milieu compatible avec la peau, les muqueuses (y compris l'intérieur des paupières et les lèvres), les ongles et/ou les fibres kératiniques (cheveux et cils).

La composition de l'invention peut contenir si nécessaire un solvant pour solubiliser le dérivé d'acide salicylique, tel que par exemple l'octyldodécanol, certains glycols, les alcools gras à chaîne courte (< C12) ou les esters à chaîne courte (< C12). La composition de l'invention est bien tolérée même en présence d'un tel solvant.

La nature de la phase huileuse rentrant dans la composition de l'émulsion H/E n'est pas critique et elle peut ainsi être constituée par tous les corps gras et notamment les huiles, classiquement utilisés dans les domaines cosmétique et/ou dermatologique.

Parmi les huiles utilisables dans l'émulsion de l'invention, on peut notamment citer par exemple les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (myristate d'isopropyle, octanoate de cétéaryle, polyisobutylène, palmitate d'éthyl-hexyle, alkyl-benzoates), les huiles de silicone (cyclométhicone telle que le cyclohexadimethylsiloxane, polydiméthylsiloxanes, polyméthylphénylsiloxanes, polydiméthylfluorosiloxanes) et les huiles fluorées. Les autres corps gras susceptibles d'être présents dans la phase huileuse peuvent être par exemple les acides gras, les alcools gras tels que l'alcool stéarylique et les cires.

La phase huileuse de l'émulsion peut représenter de 1 à 50 % et mieux de 5 à 40 % en poids par rapport au poids total de la composition.

La phase aqueuse de l'émulsion peut contenir notamment un ou plusieurs alcools et/ou polyols tels que l'éthanol, la glycérine, le butylèneglycol, l'isoprèneglycol, le propylèneglycol, le sorbitol, dans des concentrations allant préférentiellement de 1 à 20 % en poids du poids total de la composition. La phase aqueuse représente généralement de 1 à 80% et mieux de 30 à 70 % en poids par rapport au poids total de la composition.

En outre, de façon connue, les compositions de l'invention peuvent contenir des adjuvants habituels dans les domaines cosmétique et/ou dermatologique, tels que les actifs hydrophiles ou lipophiles, les conservateurs, les antioxydants, les parfums, les charges, les matières colorantes (pigments ou colorants), les filtres solaires, les solvants et encore les vésicules lipidiques. Ces adjuvants sont utilisés dans les proportions habituelles dans le domaine cosmétique ou dermatologique, et par exemple de 0,01 à 20 % du poids total de l'émulsion, et ils sont, selon leur nature, introduits dans la phase aqueuse ou dans la phase huileuse de la composition, ou encore dans des vésicules.

Bien entendu, l'homme de l'art veillera à choisir ce ou ces éventuels additifs et/ou leurs quantités de manière telle que les propriétés avantageuses attachées intrinsèquement à la composition conforme à l'invention ne soient pas, ou substantiellement pas, altérées par la ou les adjonctions envisagées.

Comme filtres solaires, on peut citer par exemple l'octocrylène (Uvinul N539 commercialisé par la société BASF), l'octyl methoxycinnamate (Parsol MCX commercialisé par la société Givaudan-Roure), le butyl methoxydibenzoylmethane (Parsol 1789 commercialisé par la société Givaudan-Roure).

Comme solvant que l'on peut utiliser dans la compositions de l'invention, on peut citer par exemple l'octyldodécanol.

Selon la fluidité de la composition que l'on souhaite obtenir, on peut y ajouter un ou plusieurs gélifiants comme les argiles, les gommes polysaccharides et leurs dérivés (gomme de xanthane, carboxyméthyl cellulose, hydroxypropyl guar), les polymères carboxyvinyliques ou carbomers, les polyacrylamides tels que celui commercialisé sous la dénomination SEPIGEL 305 par la société SEPPIC et les polymères d'acide acrylamidométhylpropanesulfonique au moins partiellement réticulés tels que le produit commercialisé sous la dénomination HOSTACERIN AMPS par la société HOECHST. Ces gélifiants sont généralement utilisés à des concentrations allant de 0,1 à 10 %, de préférence 0,1 à 5 % et mieux de 0,1 à 3 % du poids total de la composition.

Les compositions, objet de l'invention, trouvent leur application dans un grand nombre de traitements cosmétiques et/ou dermatologiques de la peau, des muqueuses et/ou des cheveux, y compris le cuir chevelu, notamment pour la protection, le soin, le nettoyage et/ou le maquillage de la peau et/ou des muqueuses, pour la protection et/ou le soin des cheveux et/ou pour le traitement thérapeutique de la peau, des cheveux et/ou des muqueuses et plus spécialement des lèvres.

Les compositions selon l'invention peuvent par exemple être utilisées dans des produits de soin ou de nettoyage pour le visage sous forme de lotions, crèmes ou laits, comme produits de maquillage (peau et lèvres) par incorporation de charges, de pigments ou de colorants, ou comme composition solaire par incorporation de filtre(s) solaire(s).

Les compositions de l'invention se sont révélées particulièrement appropriées pour le soin et/ou le maquillage de la peau et/ou des muqueuses et/ou des fibres kératiniques d'être humain, et notamment pour lutter contre les signes du vieillissement cutané et/ou pour lisser la peau du visage et/ou du corps et/ou pour traiter les rides et les ridules de la peau et/ou pour stimuler le processus de renouvellement épidermique et/ou pour traiter l'acné et/ou pour traiter les désordres cutanés. Elles permettent le traitement en douceur de la peau.

Par désordres cutanés, on entend en particulier le zona, les brûlures, l'eczéma, la démodécie, l'ulcère cutané, la fibrose, le contrôle des cicatrisations, le psoriasis, les prurits, les dermatites, l'ichtyose, les cors et les verrues.

Aussi, l'invention a encore pour objet un procédé de traitement cosmétique pour la protection, le soin, le nettoyage et/ou le maquillage de la peau et/ou des muqueuses et/ou des fibres kératiniques, consistant à appliquer sur la peau et/ou les muqueuses et/ou les fibres kératiniques, une composition telle que définie ci-dessus.

L'invention a encore pour objet un procédé de traitement cosmétique pour lutter contre les signes du vieillissement cutané et/ou améliorer l'éclat du teint et/ou lisser la peau du visage et/ou du corps et/ou traiter les rides et les ridules de la peau et/ou stimuler le processus de renouvellement épidermique, consistant à appliquer sur la peau une composition telle que définie ci-dessus.

L'invention a encore pour objet l'utilisation cosmétique de la composition telle que définie ci-dessus, pour le traitement en douceur de la peau.

L'invention a aussi pour objet l'utilisation de la composition définie ci-dessus pour la fabrication d'une composition dermatologique destinée à lutter contre les signes du vieillissement cutané et/ou à lutter contre l'acné et/ou à lutter contre les désordres cutanés.

D'autres caractéristiques et avantages de l'invention ressortiront mieux des exemples qui suivent, donnés à titre illustratif et non limitatif. Les proportions sont données en pourcentage pondéral.

### Exemple 1 : Crème

| | | |
|---|---|---|
| **A -** | Glucate SS [Amerchol] (Methyl Glucose Sesquistearate) | 2 % |
| | Alcool stéarylique | 1,5 % |
| | Cyclométhicone | 10 % |
| | Isoparaffine hydrogénée | 7 % |
| | Octocrylene (Uvinul N539) | 2 % |
| | Octyldodecanol | 5 % |
| | Capryloyl Salicylic Acid | 1 % |
| | Parfum | 0,3 % |
| | | |
| **B-** | Eau | qsp 100 % |
| | Glycérine | 5 % |
| | Glucamate SSE-20 [Amerchol] | |
| | (PEG-20 Methyl Glucose Sesquistearate) | 2 % |
| | Conservateurs | 0,6 % |
| | | |
| **C -** | Hostacerin AMPS | 1,2 % |
| | | |
| **D -** | Sepigel 305 | 1 % |

### Mode opératoire :

La phase A est chauffée à 75°C jusqu'à parfaite solubilisation ainsi que la phase B.

La phase A est introduite dans la phase B sous agitation, jusqu'à obtention d'une émulsion fine et régulière.

La phase C est ajoutée à 60°C et dispersée sous agitation, puis la phase D est introduite de la même façon. On refroidit sous agitation.

La crème obtenue utilisée en application sur la peau permet d'améliorer l'éclat du teint.

### Exemple 2 : Emulsion fluide

| | | |
|---|---|---|
| **A -** | Glucate SS [Amerchol] (Methyl Glucose Sesquistearate) | 1,5 % |
| | Alcool stéarylique | 1 % |
| | Cyclométhicone | 5 % |
| | Octocrylene (Uvinul N539) | 2 % |
| | Octyldodecanol | 3 % |
| | Capryloyl Salicylic Acid | 0,5 % |
| | Parfum | 0,2 % |
| | Conservateurs | 0,1 % |
| | | |
| **B -** | Eau | qsp 100 % |
| | Glycérine | 3 % |
| | Glucamate SSE-20 [Amerchol] | |
| | (PEG-20 Methyl Glucose Sesquistearate) | 1,5 % |
| | Conservateurs | 0,5 % |
| | | |
| **C -** | Eau | 15 % |
| | Xanthan Gum | 0,1 % |
| | | |
| **D -** | Sepigel 305 | 1 % |

### Mode opératoire :

La phase A à 75°C est introduite dans la phase B à 75° C sous agitation, jusqu'à obtention d'une émulsion fine et régulière.

La phase C est homogénéisée sous agitation dans l'eau à 50°C puis introduite sous agitation à 50°C au mélange A + B.

La phase D est introduite à 50°C et dispersée sous agitation.

Le fluide obtenu est refroidi sous agitation.

On obtient une émulsion qui par application régulière sur la peau en améliore le renouvellement cellulaire.

TEST : Un test de viabilité cellulaire sur peau reconstruite (EPISKIN TM) a été réalisé avec un support qui est un gel émulsionné à base d'hostacérin AMPS (comparatif) et avec une émulsion selon l'invention. La tolérance est d'autant meilleure que la viabilité cellulaire est grande. Les résultats de viabilité cellulaire sont donnés dans le tableau suivant :

| | **Support gel émulsionné** | | | **Emulsion Glucate/Glucamat** | | |
|---|---|---|---|---|---|---|
| ***% Capryloyl-salicylic acid*** | 0,1 | 0,3 | 0,5 | 0,1 | 0,3 | 0,5 |
| ***Viabilité Cellulaire réalisé sur EPISKIN™**** | 88,4 | 67,7 % | 34,4 % | 100 % | 100 % | 78,9 % |

Ce test montre que l'émulsion selon l'invention permet d'augmenter de façon significative la tolérance des dérivés de l'acide salicylique.

## Revendications

1. Composition cosmétique et/ou dermatologique, sous forme d'une émulsion huile-dans-eau, **caractérisée en ce qu'**elle a un pH allant de 4 à 7 et **en ce qu'**elle contient (i) de l'acide salicylique et/ou au moins un dérivé d'acide salicylique, (ii) au moins un ester d'acide gras et de glucose et/ou d'alkylglucose, et (iii) au moins un éther oxyéthyléné d'acide gras et de glucose et/ou d'alkylglucose.

2. Composition selon la revendication précédente, **caractérisée en ce que** la chaîne grasse dans l'ester d'acide gras et de (alkyl)glucose ou dans l'éther oxyéthyléné d'acide gras et de (alkyl)glucose comporte de 8 à 30 atomes de carbone.

3. Composition selon la revendication 1 ou 2, **caractérisée en ce que** l'ester d'acide gras et de glucose ou d'alkylglucose est choisi parmi les esters gras de méthylglucoside.

4. Composition selon la revendication précédente, **caractérisée en ce que** l'ester gras de méthylglucoside est choisi dans le groupe formé par le diester de méthylglucoside et d'acide oléique, l'ester mixte de méthylglucoside et du mélange acide oléique / acide hydroxystéarique, l'ester de méthylglucoside et d'acide isostéarique, l'ester de méthylglucoside et d'acide laurique, l'ester de méthylglucoside et d'acide isostéarique, le mélange de monoester et de diester de méthylglucoside et d'acide isostéarique, le mélange de monoester et de diester de méthylglucoside et d'acide stéarique et leurs mélanges.

5. Composition selon l'une quelconque des revendications précédentes, **caractérisée en ce que** la quantité d'ester(s) d'acide gras et de glucose ou d'alkylglucose va de 0,1% à 10% en poids par rapport au poids total de la composition.

6. Composition selon l'une quelconque des revendications précédentes, **caractérisée en ce que** l'éther oxyéthyléné d'acide gras et de glucose ou d'alkylglucose comporte de 10 à 100 groupements oxyéthylénés.

7. Composition selon l'une quelconque des revendications précédentes, **caractérisée en ce que** l'éther oxyéthyléné d'acide gras et de glucose ou d'alkylglucose est choisi parmi les éthers oxyéthylénés d'acide gras et de méthylglucose.

8. Composition selon la revendication précédente, **caractérisée en ce que** l'éther oxyéthyléné d'acide gras et de glucose ou d'alkylglucose est choisi dans le groupe formé par l'éther de polyéthylène glycol de diester de méthyl glucose et d'acide stéarique à environ 20 moles d'oxyde d'éthylène, l'éther de polyéthylène glycol du mélange de monoester et de diester de méthyl glucose et d'acide stéarique à environ 20 moles d'oxyde d'éthylène et leurs mélanges.

9. Composition selon l'une quelconque des revendications précédentes, **caractérisée en ce que** la quantité d'éther(s) oxyéthyléné(s) d'acide gras et de (alkyl)glucose va de 0, 1% à 10% en poids par rapport au poids total de la composition.

10. Composition selon l'une quelconque des revendications précédentes, **caractérisée en ce que** le dérivé d'acide salicylique est un dérivé de formule (I) ou d'un sel d'un tel dérivé : 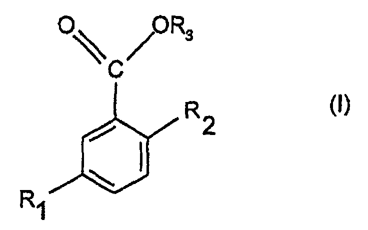 dans laquelle :
R₁ représente l'hydrogène ou une chaîne aliphatique, alcoxy, ester ou cétoxy, saturée, linéaire, ramifiée ou cyclisée, une chaîne insaturée portant une ou plusieurs doubles liaisons conjuguées ou non, ces chaînes comportant de 1 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ;
R₂ représente un groupement hydroxyle ou une fonction ester de formule (II) : 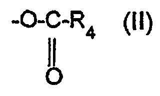 où R₄ représente un groupement aliphatique saturé ou un groupement alcényle ayant de 1 à 18 atomes de carbone ;
R₃ représente l'hydrogène ou une chaîne linéaire ou ramifiée, saturée ou insaturée, ayant de 2 à 30 atomes de carbone, comportant éventuellement un ou plusieurs substituants.

11. Composition selon l'une quelconque des revendications précédentes, **caractérisée en ce que** le dérivé d'acide salicylique est choisi parmi l'acide n-octanoyl-5-salicylique, l'acide n-décanoyl-5-salicylique, l'acide n-dodécanoyl-5-salicylique, l'acide n-octyl-5-salicylique, l'acide n-heptyloxy-5-salicylique, l'acide 5-tert-octylsalicylique, l'acide 5-butoxysalicylique, l'acide 5-éthoxysalicylique, l'acide 5-méthoxysalicylique, l'acide 5-propoxysalicylique, l'acide 5-méthylsalicylique, l'acide 5-éthylsalicylique, l'acide 5-propylsalicylique, les sels de ces acides, le salicylate de cétyle, le salicylate de dodécyle, le salicylate de tridécyle et leurs mélanges.

12. Composition selon l'une quelconque des revendications précédentes, **caractérisée en ce que** la quantité d'acide salicylique et/ou de dérivé(s) d'acide salicylique va de 0,001 à 10 % en poids par rapport au poids total de la composition.

13. Composition selon l'une quelconque des revendications précédentes, **caractérisée en ce que** la phase huileuse représente de 1 à 50 % en poids par rapport au poids total de la composition.

14. Composition selon l'une quelconque des revendications précédentes, **caractérisée en ce qu'**elle contient en outre au moins un gélifiant.

15. Procédé de traitement cosmétique pour la protection, le soin, le nettoyage et/ou le maquillage de la peau et/ou des muqueuses et/ou des fibres kératiniques, consistant à appliquer sur la peau et/ou les muqueuses et/ou les fibres kératiniques, une composition selon l'une quelconque des revendications précédentes.

16. Procédé de traitement cosmétique pour lutter contre les signes du vieillissement cutané et/ou améliorer l'éclat du teint et/ou lisser la peau du visage et/ou du corps et/ou traiter les rides et les ridules de la peau et/ou stimuler le processus de renouvellement épidermique, consistant à appliquer sur la peau une composition selon l'une quelconque des revendications 1 à 14.

17. Utilisation de la composition selon l'une quelconque des revendications 1 à 14, pour la fabrication d'une composition dermatologique destinée à lutter contre les signes du vieillissement cutané et/ou à lutter contre l'acné et/ou à lutter contre les désordres cutanés.

18. Utilisation cosmétique de la composition selon l'une quelconque des revendications 1 à 14, pour le traitement en douceur de la peau.

## Patentansprüche

1. Kosmetische und/oder dermatologische Zusammensetzung in Form einer Öl-in-Wasser-Emulsion, **dadurch gekennzeichnet, dass** sie einen pH-Wert von 4 bis 7 aufweist und dadurch, dass sie (i) Salicylsäure und/oder mindestens ein Salicylsäurederivat, (ii) mindestens einen Glucosefettsäureester und/oder Alkylglucosefettsäureester und (iii) mindestens einen ethoxylierten Ether einer Fettsäure und von Glucose und/oder Alkylglucose enthält.

2. Zusammensetzung nach dem vorhergehenden Anspruch, **dadurch gekennzeichnet, dass** die Fettkette in dem (Alkyl)glucosefettsäureester oder in dem ethoxylierten Ether einer Fettsäure und von (Alkyl)-glucose 8 bis 30 Kohlenstoffatome aufweist.

3. Zusammensetzung nach Anspruch 1 oder 2, **dadurch gekennzeichnet, dass** der Glucosefettsäureester oder Alkylglucosefettsäureester unter Methylglucosidfettsäureestern ausgewählt ist.

4. Zusammensetzung nach dem vorhergehenden Anspruch, **dadurch gekennzeichnet, dass** der Methylglucosidfettsäureester unter dem Diester von Methylglucosid und Ölsäure, dem gemischten Ester von Methylglucosid und dem Gemisch von Ölsäure und Hydroxystearinsäure, dem Ester von Methylglucosid und Isostearinsäure, dem Ester von Methylglucosid und Laurinsäure, dem Ester von Methylglucosid und Isostearinsäure, dem Gemisch des Monoesters und des Diesters von Methylglucosid und Isostearinsäure, dem Gemisch des Monoesters und des Diesters von Methylglucosid und Stearinsäure und deren Gemischen ausgewählt ist.

5. Zusammensetzung nach einem der vorhergehenden Ansprüche, **dadurch gekennzeichnet, dass** der Mengenanteil des Glucosefettsäureesters (der Glucosefettsäureester) oder Alkylglucosefettsäureesters (der Alkylglucosefettsäureester) im Bereich von 0,1 bis 10 Gew.-%, bezogen auf das Gesamtgewicht der Zusammensetzung, liegt.

6. Zusammensetzung nach einem der vorhergehenden Ansprüche, **dadurch gekennzeichnet, dass** der ethoxylierte Ether einer Fettsäure und von Glucose oder Alkylglucose 10 bis 100 ethoxylierte Gruppen aufweist.

7. Zusammensetzung nach einem der vorhergehenden Ansprüche, **dadurch gekennzeichnet, dass** der ethoxylierte Ether einer Fettsäure und von Glucose oder Alkylglucose unter den ethoxylierten Ethern einer Fettsäure und von Methylglucose ausgewählt ist.

8. Zusammensetzung nach dem vorhergehenden Anspruch, **dadurch gekennzeichnet, dass** der ethoxylierte Ether einer Fettsäure und von Glucose oder Alkylglucose unter dem Polyethylenglykolether des Diesters von Methylglucose und Stearinsäure mit etwa 20 Mol Ethylenoxid, dem Polyethylenglykolether des Gemischs des Monoesters und des Diesters von Methylglucose und Stearinsäure mit etwa 20 Mol Ethylenoxid und den Gemischen dieser Verbindungen ausgewählt ist.

9. Zusammensetzung nach einem der vorhergehenden Ansprüche, **dadurch gekennzeichnet, dass** der Mengenanteil des ethoxylierten Ethers (der ethoxylierten Ether) einer Fettsäure und von (Alkyl)glucose im Bereich von 0,1 bis 10 Gew.-%, bezogen auf das Gesamtgewicht der Zusammensetzung, liegt.

10. Zusammensetzung nach einem der vorhergehenden Ansprüche, **dadurch gekennzeichnet, dass** das Salicylsäurederivat ein Derivat der folgenden Formel (I) oder ein Salz eines derartigen Derivats ist: 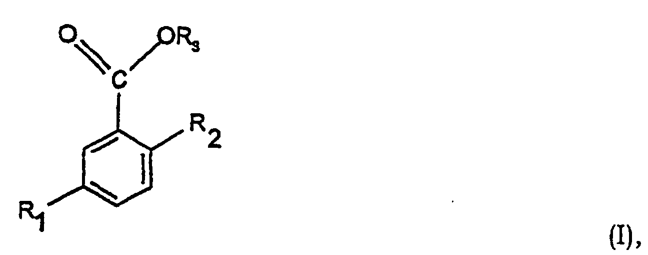 worin bedeuten:
- R₁ Wasserstoff oder eine gesättigte aliphatische Gruppe, Alkoxygruppe, Estergruppe oder Cetoxygruppe, die geradkettig, verzweigt oder cyclisch vorliegt, oder eine ungesättigte Gruppe mit einer oder mehreren gegebenenfalls konjugierten Doppelbindungen, wobei die Gruppen 1 bis 22 Kohlenstoffatome aufweisen und mit mindestens einem Substituenten substituiert sein können, der ausgewählt ist unter Halogen, Trifluormethyl, Hydroxy in freier Form oder verestert mit einer Säure, die 1 bis 6 Kohlenstoffatome aufweist, oder auch mit einer Carboxygruppe, die in freier Form oder mit einem niederen Alkohol mit 1 bis 6 Kohlenstoffatomen verestert vorliegt,
- R₂ eine Hydroxygruppe oder eine Estergruppe der Formel (II): 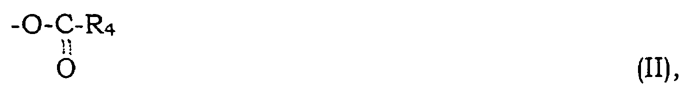 worin R₄ eine gesättigte aliphatische Gruppe oder eine Alkenylgruppe mit 1 bis 18 Kohlenstoffatomen bedeutet, und
- R₃ Wasserstoff oder eine geradkettige oder verzweigte, gesättigte oder ungesättigte Gruppe mit 2 bis 30 Kohlenstoffatomen, die gegebenenfalls einen oder mehrere Substituenten aufweist.

11. Zusammensetzung nach einem der vorhergehenden Ansprüche, **dadurch gekennzeichnet, dass** das Salicylsäurederivat unter 5-(n-Octanoyl)-salicylsäure, 5-(n-Decanoyl)-salicylsäure, 5-(n-Dodecanoyl)-salicylsäure, 5-(n-Octyl)-salicylsäure, 5-(n-Heptyloxy)-salicylsäure, 5-(*t*.-Octyl)-salicylsäure, 5-Butoxysalicylsäure, 5-Ethoxysalicylsäure, 5-Methoxysalicylsäure, 5-Propoxysalicylsäure, 5-Methylsalicylsäure, 5-Ethylsalicylsäure und 5-Propylsalicylsäure, den Salzen dieser Säuren, Cetylsalicylat, Dodecylsalicylat, Tridecylsalicylat und den Gemischen dieser Verbindungen ausgewählt ist.

12. Zusammensetzung nach einem der vorhergehenden Ansprüche, **dadurch gekennzeichnet, dass** der Mengenanteil der Salicylsäure und/oder des Salicylsäurederivats (der Salicylsäurederivate) im Bereich von 0,001 bis 10 Gew.-%, bezogen auf das Gesamtgewicht der Zusammensetzung, liegt.

13. Zusammensetzung nach einem der vorhergehenden Ansprüche, **dadurch gekennzeichnet, dass** die Ölphase 1 bis 50 Gew.-%, bezogen auf das Gesamtgewicht der Zusammensetzung, ausmacht.

14. Zusammensetzung nach einem der vorhergehenden Ansprüche, **dadurch gekennzeichnet, dass** sie ferner mindestens einen Gelbildner enthält.

15. Verfahren zur kosmetischen Behandlung zum Schutz, zur Pflege, zur Reinigung und/oder zum Schminken der Haut und/oder der Schleimhäute und/oder der Keratinfasern, das darin besteht, eine Zusammensetzung nach einem der vorhergehenden Ansprüche auf die Haut und/oder die Schleimhäute und/oder die Keratinfasern aufzutragen.

16. Verfahren zur kosmetischen Behandlung, um die Zeichen der Hautalterung zu bekämpfen und/oder den Teint strahlender zu machen und/oder die Haut des Gesichts und/oder des Körpers zu glätten und/oder Falten und Fältchen der Haut zu behandeln und/oder den Erneuerungsprozess der Epidermis anzuregen, das darin besteht, eine Zusammensetzung nach einem der Ansprüche 1 bis 14 auf die Haut aufzutragen.

17. Verwendung der Zusammensetzung nach einem der Ansprüche 1 bis 14 zur Herstellung einer dermatologischen Zusammensetzung, die zur Bekämpfung der Zeichen der Hautalterung und/oder zur Bekämpfung von Akne und/oder zur Bekämpfung von Hautkrankheiten vorgesehen ist.

18. Kosmetische Verwendung der Zusammensetzung nach einem der Ansprüche 1 bis 14 zu sanften Behandlung der Haut.

## Claims

1. Cosmetic and/or dermatological composition in the form of an oil-in-water emulsion, **characterized in that** it has a pH ranging from 4 to 7 and **in that** it contains (i) salicylic acid and/or at least one salicylic acid derivative, (ii) at least one ester of fatty acid and of glucose and/or of alkylglucose, and (iii) at least one oxyethylenated ether of fatty acid and of glucose and/or of alkylglucose.

2. Composition according to the preceding claim, **characterized in that** the fatty chain in the ester of fatty acid and of (alkyl)glucose or in the oxyethylenated ether of fatty acid and of (alkyl)glucose contains from 8 to 30 carbon atoms.

3. Composition according to Claim 1 or 2, **characterized in that** the ester of fatty acid and of glucose or of alkylglucose is chosen from the fatty esters of methylglucoside.

4. Composition according to the preceding claim, **characterized in that** the fatty ester of methylglucoside is chosen from the group consisting of the diester of methylglucoside and of oleic acid; the mixed ester of methylglucoside and of the oleic acid/hydroxystearic acid mixture; the ester of methylglucoside and of isostearic acid; the ester of methylglucoside and of lauric acid; the ester of methylglucoside and of isostearic acid; the mixture of monoester and of diester of methylglucoside and of isostearic acid; the mixture of monoester and of diester of methylglucoside and of stearic acid and mixtures thereof.

5. Composition according to any one of the preceding claims, **characterized in that** the quantity of ester(s) of fatty acid and of glucose or of alkylglucose ranges from 0.1% to 10% by weight relative to the total weight of the composition.

6. Composition according to any one of the preceding claims, **characterized in that** the oxyethylenated ether of fatty acid and of glucose or of alkylglucose contains from 10 to 100 oxyethylenated groups.

7. Composition according to any one of the preceding claims, **characterized in that** the oxyethylenated ether of fatty acid and of glucose or of alkylglucose is chosen from the oxyethylenated ethers of fatty acid and of methylglucose.

8. Composition according to the preceding claim, **characterized in that** the oxyethylenated ether of fatty acid and of glucose or of alkylglucose is chosen from the group consisting of the polyethylene glycol ether of diester of methylglucose and of stearic acid containing about 20 moles of ethylene oxide, the polyethylene glycol ether of the mixture of monoester and of diester of methylglucose and of stearic acid containing about 20 moles of ethylene oxide and mixtures thereof.

9. Composition according to any one of the preceding claims, **characterized in that** the quantity of oxyethylenated ether(s) of fatty acid and of (alkyl)glucose ranges from 0.1% to 10% by weight relative to the total weight of the composition.

10. Composition according to any one of the preceding claims, **characterized in that** the salicylic acid derivative is a derivative of formula (I) or a salt of such a derivative: 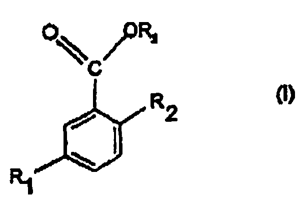 in which:
R₁ represents hydrogen or a saturated, linear, branched or cyclized aliphatic, alkoxy, ester or ketoxy chain, an unsaturated chain carrying one or more conjugated or unconjugated double bonds, these chains containing from 1 to 22 carbon atoms and being capable of being substituted with at least one substituent chosen from halogen atoms, the trifluoromethyl group, the hydroxyl groups in a free form or esterified by an acid having from 1 to 6 carbon atoms or alternatively by a carboxyl function, which is free or esterified by a lower alcohol having from 1 to 6 carbon atoms;
R₂ represents a hydroxyl group or an ester function of formula (II): 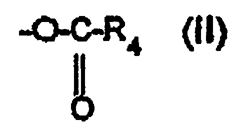 where R₄ represents a saturated aliphatic group or an alkenyl group having from 1 to 18 carbon atoms;
R₃ represents hydrogen or a saturated or unsaturated linear or branched chain having from 2 to 30 carbon atoms, optionally containing one or more substituents.

11. Composition according to any one of the preceding claims, **characterized in that** the salicylic acid derivative is chosen from 5-n-octanoylsalicylic acid, 5-n-decanoylsalicylic acid, 5-n-dodecanoylsalicylic acid, 5-n-octylsalicylic acid, 5-n-heptyloxysalicylic acid, 5-tert-octylsalicylic acid, 5-butoxysalicylic acid, 5-ethoxysalicylic acid, 5-methoxysalicylic acid, 5-propoxysalicylic acid, 5-methylsalicylic acid, 5-ethylsalicylic acid, 5-propylsalicylic acid, the salts of these acids, cetyl salicylate, dodecyl salicylate, tridecyl salicylate and mixtures thereof.

12. Composition according to any one of the preceding claims, **characterized in that** the quantity of salicylic acid and/or of salicylic acid derivative(s) ranges from 0.001 to 10% by weight relative to the total weight of the composition.

13. Composition according to any one of the preceding claims, **characterized in that** the oily phase represents from 1 to 50% by weight relative to the total weight of the composition.

14. Composition according to any one of the preceding claims, **characterized in that** it contains, in addition, at least one gelling agent.

15. Method of cosmetic treatment for the protection, care and cleansing of and/or as make-up for the skin and/or the mucous membranes and/or of the keratinous fibres, consisting in applying to the skin and/or the mucous membranes and/or the keratinous fibres a composition according to any one of the preceding claims.

16. Method of cosmetic treatment for combating the signs of skin ageing and/or for enhancing the brightness of the complexion and/or for making the skin of the face and/or of the body smooth and/or for treating the wrinkles and fine lines of the skin and/or for stimulating the process of epidermal renewal, consisting in applying to the skin a composition according to any one of Claims 1 to 14.

17. Use of the composition according to any one of Claims 1 to 14, for the manufacture of a dermatological composition intended for combating the signs of skin ageing and/or for combating acne and/or for combating skin disorders.

18. Cosmetic use of the composition according to any one of Claims 1 to 14, for gentle treatment of the skin.
